# EUROPEAN PATENT SPECIFICATION

(11) **EP 1 294 272 B1**
(45) Date of publication and mention of the grant of the patent: **15.08.2007**
(21) Application number: 01938508.7
(22) Date of filing: 22.06.2001
(51) Int. Cl.: A61B 5/00, G06F 19/00

(54) **SYSTEM FOR MAINTENANCE AND MANAGEMENT OF HEALTH**
SYSTEM ZUR GESUNDHEITSÜBERWACHUNG
SYSTEME D'ENTRETIEN ET DE GESTION DE LA SANTE

(30) Priority: 22.06.2000 WO PCT/IB00/00838
(43) Date of publication of application: 26.03.2003
(73) Proprietor: Voegeli, Fridolin, 8800 Thalwil (CH); Mindermann, Frederick J., Brentwood, TN 37027 (US)
(72) Inventor: Voegeli, Fridolin, 8800 Thalwil (CH); Mindermann, Frederick J., Brentwood, TN 37027 (US)
(74) Representative: Spierenburg, Pieter
(86) International application number: PCT/IB2001/001110
(87) International publication number: WO 2001/097686

(56) References cited:
- WO-A-00/28459
- US-A- 5 544 661

## Description

### FIELD OF THE INVENTION

The present invention relates generally to systems for maintenance and management of health for individual patients with Communication Systems for remote monitoring and controlling of large collections of individuals, persons or devices, and to Artificial Intelligence for managing and maintaining their good status and trouble-free operation.

### BACKGROUND OF THE INVENTION

In most western societies, namely in the United States and Europe, more than 30% of the population do have serious chronic health problems that challenge them in any aspects of their daily work life, and even more and worse when they leave their home to go on travel, or try to do some sports for their health. The annual medical cost in the U.S. have soared to more than 10'000 $ per non-healthy person. To bring down and control these costs, home healthcare and preventive healthcare have been launched and promoted by insurers and politicians, delivered by mostly small local care givers spread out over the country. The success of these programs was expected to be significant, especially when modern tools and methods of communication were designed, as described in earlier patents for different, interactive and interesting hardware systems and electronic data processing or EDP methods.

But all these efforts ran against the fact that life expectancy climbed faster than any corrective measures. Sooner or later it will add all of us to the group of persons who have to be constantly monitored. In these modern societies we mostly live single on our own with no mate in the home. And any retired people's home will only accept us, if we can still move around freely and do not have to stay and to be served in the bed all the time, because the next weakness or dizziness can not be predicted or immediately assessed and reacted upon.

Many tele-medical systems have been designed and described in the very details, but have not really been used wide-spread and have not contributed to successful optimization of cost and services. Main reason for this is the limited application of existing technology in hardware and software by the medical caregivers and therefore only a limited range of services are conceived and delivered.

E.g. in US-A-5,544,661 a patient monitoring system is described which includes a portable device and a central station. The portable device includes an ECG and a photo-plethysmograph connected to a patient; an arrhythmia analysis apparatus; an expert system for determining if a pre-established critical parameter set has been exceeded; and a wireless wide area communication device for automatically contacting the central station via a public cellular phone network when the critical parameter set has been exceeded. When the central station is contacted, the patient's ECG waveforms, measurements, and trends, are sent to the central monitoring station and a two way voice channel between the patient and the central station is automatically opened. The central station includes a computerized facility which has a station from which a clinician can observe the real time data being sent from the patient, the patient's historical records and from which the clinician can talk to the patient and activate therapeutic devices attached to the patient such as an external defibrillator, a pacer or an automatic drug infusion device.

The above mentioned monitoring system is provided only for emergency cases to provide some immediate action on the patient. However, the system does not give a complete overview of the patient's health plan as is normally performed by the family doctor.

In WO-A-00/28459 is described a method and computer apparatus for automated medical outcomes data feedback, medical records integration, and healthcare provider response. The method is in general directed to the transfer per e-mail of any questions to the patient which are stored in patient data log and which are transferred to any healthcare provider, if appropriate. From the expert system task lists for the appropriate actors are provided, which tasks are performed by the actors themselves.

In this document only the patient is interrogated about the outcome of any medical intervention which is a help for the medical personnel. However, this method is not a system for maintenance and management of health care as intended by the present invention.

### SUMMARY OF THE INVENTION

Accordingly, an object of the invention is a system for the maintenance and management of health of a large number of individual patients which permits an easy and complete access to all important data of the patient to be treated.

This object of the invention is accomplished by a system with the features of claim 1 and by a computer program product with the features of claim 8.

The main advantage of the system according to the present invention is that all important data can be retrieved immediately at any place. So the caregivers like medical doctors, first-aid men and emergency services can immediately decide which treatment or therapy should be given.

In systems used for the management of large industrial plants, e.g. to control and maintain large oil refineries, or systems for monitoring remote pump stations along pipelines latest sensor technology with high-speed communications and extensive Artificial Intelligence in the central supervision stations, are known for over 20 years. Repair and preventive maintenance cost have been reduced to lowest optimized levels, while at the same time availability of the very complex plants was pushed up and can be guaranteed today to 100% over 30 years.

It was surprisingly found that the functions to be implemented in efficiency optimized health maintenance of large groups of individuals, spread over rural countryside are very similar to above mentioned management systems, that secure the safe and efficient operation of all the installations in a large metropolitan subway system. The medical maintenance system according to the present invention now combines the advantages of usual medical sensors and well-trained medical personnel in hospitals, doctor's offices, wide-spread walk-in stores and emergency services with the leading technical maintenance intelligence, with the most efficient and most widely used, traditional communication tool, the telephone.

These and other advantages of the invention are disclosed in the dependent claims and the following description in which an exemplified embodiment of the invention is described with respect to the accompanying drawings.

### BRIEF DESCRIPTION OF THE DRAWINGS

- Fig. 1: shows an overview of the elements of the present invention,
- Fig. 2: shows a basic program module for monitoring and supervising,
- Fig. 3: shows another module for assessing and decision making, and
- Fig. 4: shows several medical sensors connected to a mobile telephone for communication with the maintenance and management system.

### DETAILED DESCRIPTION OF ILLUSTRATIVE EMBODIMENTS

In the following description we define as 'Patients', 'Individuals' and 'Units' the individuals and the large number of distributed entities to be monitored, managed and maintained. They all have as a main property their 'Condition', defined by the physiological data and measured through different parameters. We call 'Walk-ins' the devices, doctor's offices and labs, that measure and collect the 'Condition' data from the 'Patients' by 'Sensors' and feed the measured data into the management system.
We call 'Managers' the programs and 'Supervisors' and 'Caregivers' the persons who are in charge of the 'Condition' monitoring, like doctors, first-aid men and emergency services, which react on 'Events' defined as alarms, problems and the like. We call 'Resources' all the man hours, money, people, devices, energy and consumables or disposables used and spent for treatment, troubleshooting, assistance and for teaching and maintenance of the 'Units'.
We call 'Logs' all the electronically organized and recorded inputs of measurements, actions, notes and results. And finally we call 'Reports' all the information gathered over the whole time and compiled on different purposes to satisfy general and specific information needs, from administrative functions to strategic fact-finding and decision-making.

### MTMM System Overview

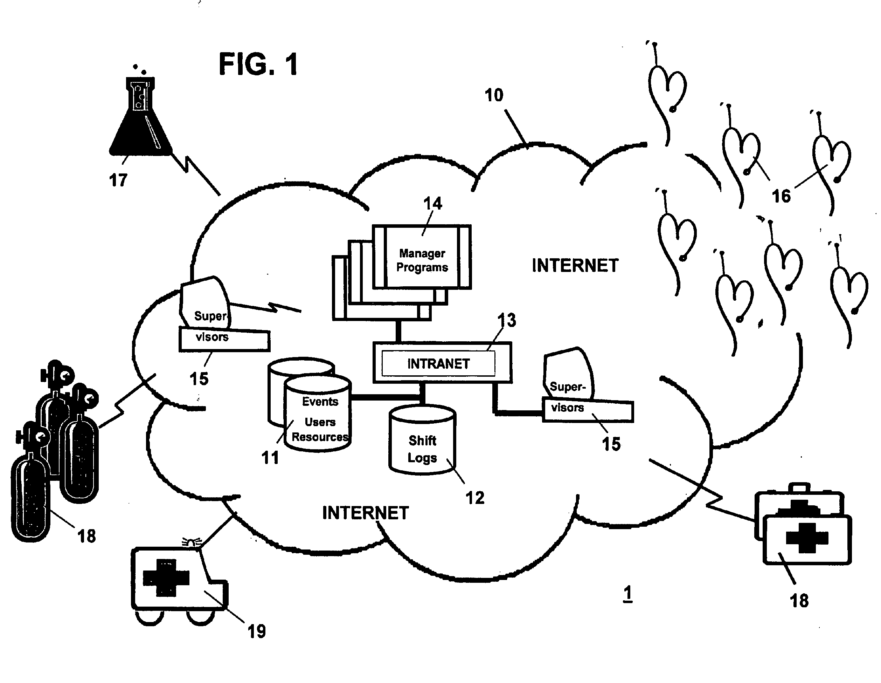

In Figure 1 a platform of the Mobile Tele-Medical Maintenance System (MTMM) is depicted, which is a computer system 1 with a combination of proven technology process control modules, hardware and software, with a known medical knowledge base, linked via the Internet 10 and with the telephone as communication net as backbone to the distributed peripheral entities. The system kernel consists of several large databases 11 containing all the relevant data of all Patients and Resources : A 'Personal Medical Web Site' contains the data in form of an EMR ('Electronic Medical Record') which is stored in a Condition Log in one of the databases 11. At the other hand the access to the EMR can be obtained via an Internet link. The system kernel resides normally on web servers and is linked to a large number of peripheral units on the individual patients.

A 'Personal Health Plan' shows all the doctor's prescriptions and recommendations to the Patient for medication, eating, physical activities, lifestyle, telephone calls to the doctor's office, etc. and the selected Condition monitoring with threshold values or limits and indications for corrective actions to caregivers should any values or limits be exceeded.

A further database 12 collects all the Condition measurements made and Event messages received, together with all the corrective actions or reactions taken and initiated by the Manager programs and the Supervisors and Caregivers, and links them to Cases, Users and Resources in a Shift Log. Also the Personal Health Plan is linked to the Shift Log.

The databases 11 and 12 are connected by and communicate over an internal network or intranet 13 with each other, directed by Manager modules 14 and Supervisors and Caregivers 15.

The kernel is surrounded by the peripherals, linked to the databases and the Manager Modules via the Internet: All the Patients, shown as 'Mobile Hearts' 16, the Walk-ins 17, the Caregivers 18 and emergency services 19. These peripherals have many ways to enter their Condition data into the databases, using e.g. smart Sensors, lab instruments and PC's, alarm buttons and help-phones, or just 'manual' typing or voice input in plain text on a terminal. Also scanners can be used to enter handwritten information, which can be transferred to machine-readable information by a known transposing program, into the databases 11.

### Log Manager Module

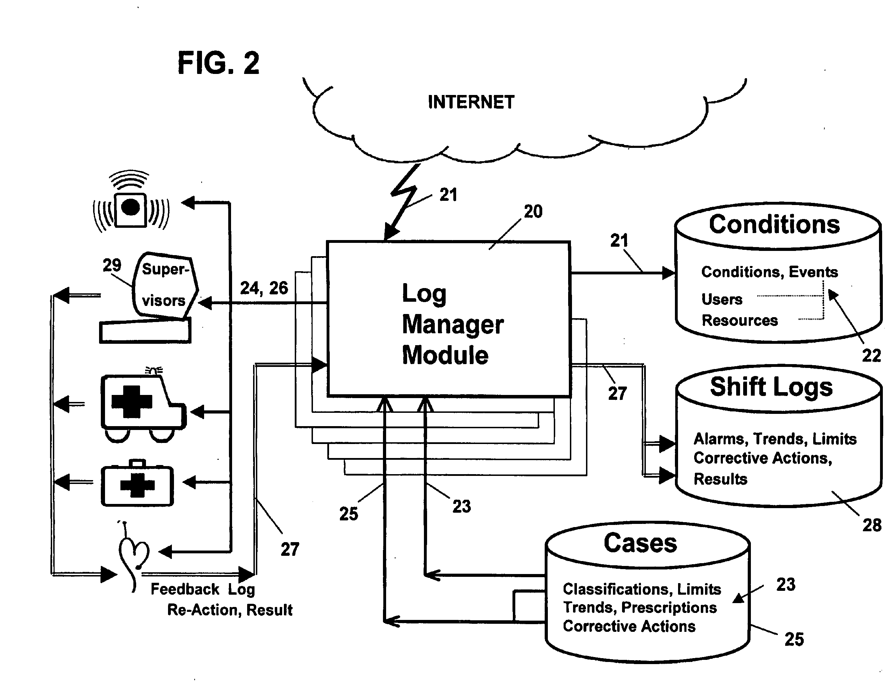

In Figure 2 a Log Manager Module 20 is shown, which is the basic program module of the MTMM system, doing Condition Monitoring and Supervision. All Conditions, i.e. measurements, alarms and other inputs, and Events 21 are entered and logged into the databases. All the necessary links 22 to Personal Health Plans are updated, enabling fast retrieval and augmentation of knowledge stored in a knowledge database.

The Log Manager Module as a condition monitoring device assesses the data entered and, if preset limits or trends 23 have been exceeded, automatically generates alarms 24 to Supervisors, Caregivers, Patients, emergency services and other medical personnel on duty. Based on the doctors prescriptions 25 and case classifications 23 the Log Manager Module initiates calls by doctors, visits by Caregivers, medication changes by pharmacies, device exchange and maintenance by technicians, etc.

The Log Manager Module sends the orders 26 for these corrective actions also to the human shift Supervisor who may then decide on priorities and may coordinate the actions. The Log Manager Module keeps track of all the related events, of his own actions and initiations, of the Supervisors decisions, of the actions and the results feedback 27, in a fully integrated Shift Log 28.

It is a main characteristic of the MTMM system that all the tasks can be and some have to be executed in parallel by the software Manager Modules and the human Supervisors.

The Log Manager Module is an excellent tool for any shift supervision 29, also for medical supervision. No handwritten notes are necessary, all important events are always present and can be called upon directly on-screen. The planning of shifts in care giving, in emergency telephone services, for ambulances and for hospitals, etc. becomes "easy" to manage.

### Diagnostic Manager Module

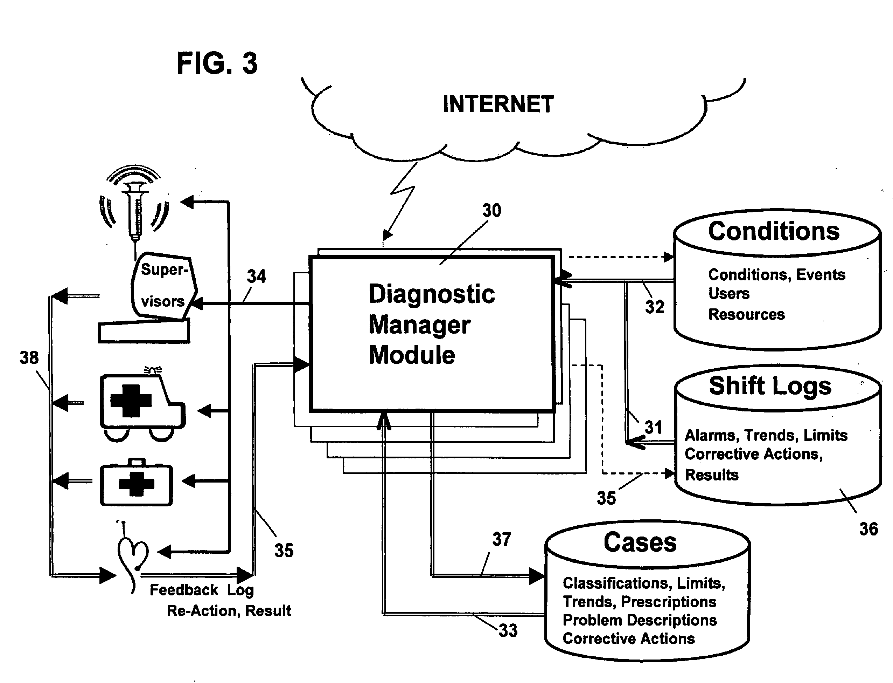

In Figure 3 a Diagnostic Manager Module 30 as an evaluating device is shown, which is a 'knowledge driven brain' of the MTMM system, doing the assessment of Conditions and Events, taking decisions and making recommendations to the Caregivers and the Patients for troubleshooting and treatment and keeping record of the results 35 of each action.

In response to alarms 31, Condition measurements 32 and descriptions 33 of problem situations, the Diagnostic Manager Module enables Supervisors, Caregivers, emergency technicians and Patients to assess the situation 34 and to identify the appropriate corrective action quickly and accurately. This module, applying usual software technologies, combines actual 'best-practice' diagnosis guidelines with a unique knowledge base from the electronic Shift Logs 36, learning and acquiring new knowledge 37 continuously.

With this knowledge always recallable from the Diagnostic Manager, all the Supervisors, Caregivers, emergency and shift staff are learning continuously on their job 38, reducing cost of training significantly and improving the quality of their service. Internet-based teaching can be included, linked and added to the recommended procedures.

### Health Plan Manager Module

This is the first program in the 'Maintenance Control Cycle' and it enables the Caregivers to tailor-made their 'Teaching and Treatment' service to the Patients' individual needs, while optimizing the deployment of their resources and reduce the cost generated. But it is also the follow-up program that enables the Caregiver and the Patient to adjust their efforts and to optimize the deviation of the Condition from 'best practice' accepted standards for the Patient's sex, age, population group, job, education, activity, etc.

All Teaching, Treatment, medications, activities, Condition monitoring parameters with devices and actions are managed and displayed to Caregiver and Patient in a Health Plan (or Disease Management) classification system. All the different sources of services (doctors, walk-ins, care givers visits) and their care and assistance given, planned and unplanned, are selected and implemented in this 'Personal Health Plan'. For this task the Health Plan Manager Module bases on the Caregivers experience and 'best practice' guidelines but also on all company internal rules and ISO certified standards.

The 'Personal Health Plan' keeps the overall picture of all the service efforts done and the resulting Condition, summarized into an integral history of the Patient's health. Transitions in his 'medical life', by going to the hospital, being released to a nursing home, moving to another area and another family doctor, changing job and health insurance, etc. can be managed without transferring the patients data in a old-fashioned, cumbersome way. In the MTMM system the 'Mobile Patient' can really leave home 'without it' and access his 'Private Patient Web site' and his 'Personal Health Plan' from any place in this world, while still being monitored, supervised and assisted like in his home town.

### Service Manager Module

This is the module for the Caregiver's logistics organization. It enables them to monitor and optimize their highly complex, distributed and costly operations. It manages the collection of all service contracts and doctors prescriptions. It controls all preventive care and unscheduled troubleshooting events, and includes them into a "integral" care cycle. It allows regular preventive care work to be scheduled, planned, ordered and monitored down to the smallest consumable needed.

All services, all devices and Resources are recorded and managed in a parts classification system. This system includes all necessary information about suppliers, contracts, equipment and warranties. The same way all the patients and the care, planned and unplanned, are managed in the Health Plan Manager, the same way all peripheral entities, Devices and Resources, are maintained ready and in good condition. For this purpose the Service Manager Module also contains all the information according to ISO standards and company internal rules and specs.

While the care givers work is performed, all data about type of service, problems solved, how, with which medication and material and at what cost, are entered and a special Care Service Report is generated. Findings, conclusions and actions taken are then transferred to the Diagnostic Manager's knowledge data base.

### Report Manager Module

This is the evaluation and analysis program for the management of this fully integrated Health Maintenance Process. It enables continuous and periodical monitoring and classification of the activities in the electronic Shift Log, in the service orders and in other Events. This makes transparent the patients and population group's proneness to problems just as well as the cost control, hours spent and medication and consumables used.

The Report Manager Module enables monitoring the compliance of the therapies implemented and the outcome of the therapies administered. It therefore delivers the basis to all measures to improve efficiency and quality of the Care-giving process ('Teaching and Treating') throughout the whole service industry and its market.

All the data bases are of the relational type, based on standard software. It therefore is easy to add report generators to create additional, custom made reports, as well as to implement interfaces to sophisticated Management Information Systems.

### Peripherals Integration

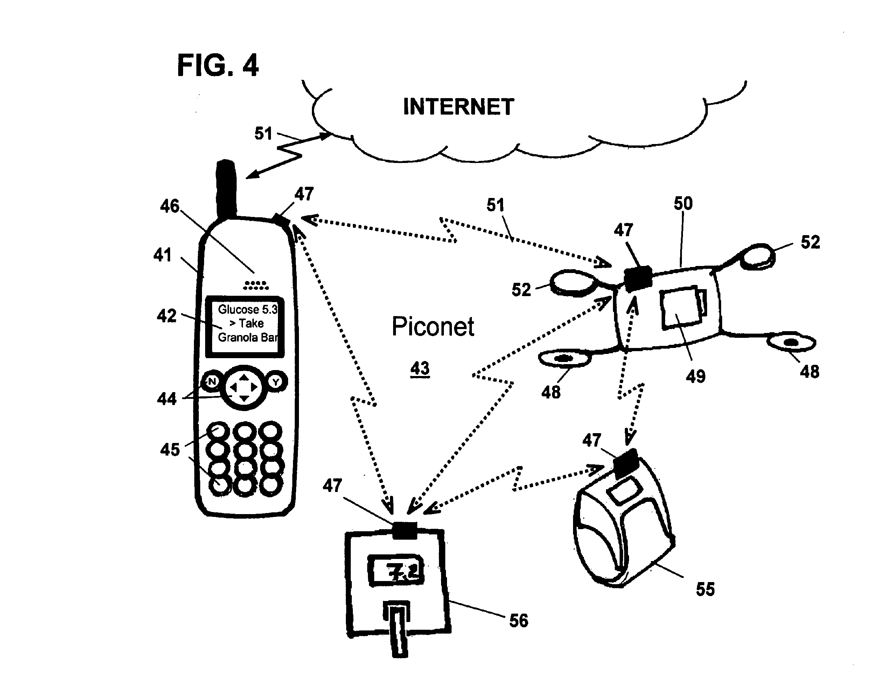

In Figure 4 the integration of peripherals is depicted. The MTMM system uses the open architecture of Internet, telephone and the so called 'RF-Piconet', which finally enables a full integration of all types of peripherals via the easy to use, omnipresent telephone link, by wire and wireless.

The telephones, mainly the new mobile phones (also built into other devices, like watches, palm PC's), shall become the favorites in personal medical condition monitoring. Installing and assigning new peripherals is easy: New users get their phone with a world-wide private number (= device address). They can use it where ever they are and what ever telecom company's infrastructure they are based on.
The new mobile phones have become real Internet terminals, with which huge amounts of information can be downloaded and displayed, if necessary. They are used in the MTMM system as hubs for the very small network of different intelligent Sensors on the Patients body and devices nearby in the home, the "Piconet" 43. The mobile phone can poll Condition data from the Sensors, display them, pre-assess them and transmit them on to the databases in the kernel. And all Users have been comfortable for a long time with the user-friendly guidance "if you need assistance, press 0" and will also get used to high-tech voice input and voice recognition.

In most urban areas the mobile phone system enables tracking of the Patient's position down to some 50 m accuracy, an ideal tool for any Emergency Medical Service but also for caring friends and family members.

The Sensors have become 'intelligent' and can communicate over the same Internet with the central Log Manager Module. In the MTMM system they download requests and limits for bio-signal acquisition and transmission and do the Condition monitoring. They can do basic pre-assessments of the bio-signals and can give local alarms to the Patient, if limits are exceeded. They integrate RF-Piconet transceivers (like Bluetooth) that enable them to start communication with any other device, when ever they are switched on.

The MTMM system performs thus the functions of "CSACT" : **C**ondition monitoring, **S**upervision, **A**ssessment, **C**ommunication, **T**reatment and **T**eaching. It applies different methods of signal analysis in the intelligent sensors and in the kernel to assess the actual status of the individuals, it communicates the assessment to diagnosing programs and supervisory personnel and commands the actuators, assistance personnel and the individual to treat itself and to cope with the actual situation. Using standard and Artificial Intelligence software the MTMM system will do : Alarms, immediate corrective actions, reports, logs including accounting; recalls, to care providers, optimized plans for preventive check-ups and care; optimized management of all personnel, material, devices and infrastructure resources of the care givers; all sorts of statistics for any level of the medical hierarchy in politics, public and private health organizations and insurers.

Fig. 4 shows such a typical 'Piconet' 43 in the MTMM system: a mobile telephone 41 with its display 42 and special display control buttons 44, its numeric keypad 45 in order to type in values from stand-alone tests, speaker and microphone for voice output and input 46. The mobile telephone 41 communicates with any server on the Internet and its built-in RF-Piconet transceiver 47 communicate with two Sensors on the Patients body, and with one tabletop device.

A heart monitor 50 senses the ECG with two electrodes 48, pre-analyzes it and stores it on a memory-card 49. It also monitors the breathing rate and breathing volume by breath sensors 52. The measured values-are stored and checked for coherence between two normally independent signals. The heart monitor 50 uses the display 42 of the mobile telephone 41 to show to the patient locally his Condition with heart-rate, body temperature, breath rate and breath volume. The mobile telephone 41 transmits further the Condition data over the Internet 53 to the MTMM system and to its Log Manager Module. The heart monitor 50 and mobile telephone 41 may communicate continuously or periodically with each other, or only in case of alarms or exceeding of limits.

A non-invasive blood pressure unit 55 is used only a few times a day, according to the doctor's request. So are many other devices; when ever they are taken out of the pocket and switched on, they automatically search over transceiver 47 the area for an active 'Piconet' to hook on. The RF-Piconet transceiver 47 (like Bluetooth) offers this type of network, like the wire-bound Ethernet.

A glucose meter 56 might be of the old fashioned strip type. With a built-in RF-Piconet transceiver 47 it will automatically call the MTMM system via the mobile telephone's access to the Internet 51 and will ask for Condition measurement schedules. If it does not have the network capability, the MTMM system will request the Patient via the mobile telephone's display 42 to do a measurement at the prescribed time and to type in the resulting value via the numeric keypad 45 of the mobile telephone 41.

It should be clear to the skilled person that instead of mobile telephones 41 also normal telephone sets or PC's with a monitor can be used.

Built-in data conversion modules in the Log Manager Module allow defined alarm and condition data formats to be transferred directly into the database. The Supervisors can assess the individual patients status 'real-time' by calling their mobile phone's "Pico-net" hub.

But also the devices leased and maintained by the Caregiver, by the ambulances and by medical personnel can be called and directed in 'real-time'. The logs of their deployment at the same time are transferred to commercial bookkeeping and invoicing systems within the company offering useful and efficient support not found in other systems.

### Standard Hardware and Software

The system is standard-based, running on Windows and Unix platforms. The databases are all relational. The system is also modular. The individual modules can be used independently of one another.

The favorite network, fully exploiting the new WAP (Wireless Application Protocol) capabilities, is the worldwide Internet, or an Intranet in large companies, hospitals, nursing home holdings, etc. The mobile telephone can control several sensors : and input devices. Bluetooth is the future standard in these "Piconets".

## Claims

1. A system for maintenance and management of health care for a large number of individual patients comprising a computer system and a communication system connected in communicating relationship to the computer system, wherein
the computer system (1) comprises
- a condition monitoring device (20) comprising a Condition Log (11; 22), provided for entering and storing all important physiological data and event information of each individual patient together with alarm signals for said individual patient,
- a supervision device (20) comprising a Shift Log (12; 28), provided for assessing the alarm signals and event information and for transmitting the alarm signals and event information to care givers involved in order to initiate a corrective action on each individual patient and for requesting a report about the corrective action and its results, and for storing the reports, and
- an evaluating device (30) provided for evaluating problems and the patterns of the measurements by comparing the problems and patterns with accumulated information in a knowledge database (36), for selecting appropriate corrective action to be recommended to the caregivers and for requesting a report of the caregivers about the corrective action and its results, and for entering the reports into the knowledge database, thereby accumulating and enlarging the knowledge database, and
- the communication system (10; 13; 43; 51) is provided for transmitting alarms and recommendations to a supervisor, in order to correct and/or release the alarms and recommendations, being transmitted further to the caregivers for taking the recommended action to the individual patient.

2. A system according to Claim 1, whereas the communication system (10'; 13; 43; 51) comprises devices for Internet access, especially Internet accessible mobile telephones (41), PDA's and PC's.

3. A system according to Claim 1 or 2, wherein sensors (48; 50; 52; 55; 56) are provided for measuring the physiological data on the patient which are connected to the communication system (10; 13; 43; 51).

4. A system according to one of Claims 1 to 3, comprising a Medical Web Site stored in the computer system (1), which contains a separate database containing all medical data and the medical history of each individual patient, which is accessible by express authorization of the individual patient and/or his caregiver only.

5. A system according to one of Claims 1 to 4 comprising a Personal Health Plan stored in the computer system (1), containing the doctor's prescriptions for medication, treatments, practicing and lifestyle of the individual patient, a schedule for the monitoring of conditions stored in the Condition Log, the selection of sensors to be used, the threshold values of the physiological data to be monitored and corrective actions, whereas the monitored data, compliance for therapies and the outcome of the therapies are attached to the Plan, which is accessible by express authorization of the individual patient and/or his caregiver only.

6. A system according to one of Claims 1 to 5 comprising a Log Manager Module (20) for monitoring the alarm signals and the event information in the Condition Log (11; 22) and for supervising the reports in the Shift Log (12; 28; 36).

7. A system according to one of Claims 1 to 6, comprising a Diagnostic Manager Module (30) for diagnosing the condition of each individual patient by means of comparing data stored in expert computer programs containing diagnosis guidelines with the accumulated data of the knowledge database.

8. A computer program product stored on a medium usable in a computer system for maintenance and management of health care for a large number of individual patients, comprising software code adopted to cause the system according to Claim 1 to perform the following steps:
- transmitting, entering and storing all important physiological data and event information of each individual patient together with alarm signals for said individual patient into a Condition Log (11; 22) of a condition monitoring device (20),
- assessing the alarm signals and event information and transmitting the alarm signals and event information to care givers involved in order to initiate a corrective action on the patient and requesting a report about the corrective action and its results, and storing the reports into a Shift Log (12; 28) of a supervision device (20), and
- evaluating problems and the patterns of the measurements by comparing the problems and patterns, with accumulated information in a knowledge database (36) of an evaluating device (30), selecting appropriate corrective action to be recommended to the caregivers and for requesting a report of the caregivers about the corrective action and its results, and entering the reports into the knowledge database, whereby accumulating and enlarging the knowledge database, and
- transmitting alarms and recommendations by means of a communication system (10; 13; 43; 51) connected to the computer system (1) to a supervisor, who corrects and/or releases the alarms and recommendations, being transmitted further to the caregivers for taking the recommended action.

## Patentansprüche

1. System zur Verwaltung und zum Management der Gesundheitsversorgung einer großen Anzahl einzelner Patienten, das ein Computersystem und ein mit dem Computersystem in Kommunikationsverbindung stehendes Kommunikationssystem umfasst,
wobei das Computersystem (1) Folgendes umfasst:
- eine Zustandskontrollvorrichtung (20), die ein Zustands-Protokoll (11; 22) umfasst, zum Eingeben und Speichern aller wichtigen physiologischen Daten und Ereignisinformationen der einzelnen Patienten, gemeinsam mit Warnsignalen für den einzelnen Patienten,
- eine Supervisionsvorrichtung (20), die ein Übergabe-Protokoll (12; 28) umfasst, zum Bewerten der Warnsignalen und Ereignisinformationen und zum Übertragen der Warnsignalen und Ereignisinformationen an die beteiligten Pflegepersonen, mit dem Ziel, für den einzelnen Patienten Korrekturmaßnahmen zu ergreifen und zur Abfrage eines Berichts über die Korrekturmaßnahme und deren Ergebnisse sowie zum Speichern der Berichte, und
- eine Evaluierungsvorrichtung (30) zum Evaluieren von Problemen und der Messwertmuster mittels Vergleichens der Probleme und Muster mit den akkumulierten Informationen in einer Wissensdatenbank (36), zur Auswahl geeigneter Korrekturmaßnahmen, die den Pflegepersonen empfohlen werden, und zur Anfrage eines Berichts der Pflegepersonen über die Korrekturmaßnahmen und deren Ergebnisse sowie zum Eingeben der Berichte in die Wissensdatenbank, wodurch die Wissensdatenbank akkumuliert und vergrößert wird, und
- wobei das Kommunikationssystem (10; 13; 43; 51) zur Übertragung von Warnungen und Empfehlungen an einen Supervisor vorgesehen ist, um die Warnungen und Empfehlungen zu korrigieren und/oder abzugeben, die weiter an die Pflegepersonen geleitet werden, damit diese an den einzelnen Patienten die empfohlenen Maßnahmen vornehmen können.

2. System gemäß Anspruch 1, wobei das Kommunikationssystem (10'; 13; 43; 51) Vorrichtungen für den Internetzugang besitzt, insbesondere Internetfähige Mobiltelefone (41), PDAs und PCs.

3. System gemäß Anspruch 1 oder 2, wobei Sensoren (48; 50; 52; 55; 56) zum Messen der physiologischen Daten am Patienten vorgesehen sind, die mit dem Kommunikationssystem (10; 13; 43; 51) verbunden sind.

4. System gemäß einem der Ansprüche 1 bis 3, das eine im Computersystem (1) gespeicherte Medizinische Website umfasst, die eine getrennte Datenbank enthält, in der alle medizinischen Daten und die medizinische Geschichte jedes einzelnen Patienten enthalten sind, welche nur durch ausdrückliche Autorisierung des einzelnen Patienten und/oder seiner Pflegeperson zugreifbar ist.

5. System gemäß einem der Ansprüche 1 bis 4, das einen im Computersystem (1) gespeicherten Persönlichen Gesundheitsplan umfasst, in dem die ärztlichen Verschreibungen zur Medikation, Behandlungen, Praxis und Lebensstil des einzelnen Patienten, ein Zeitplan für die Kontrolle der im Zustandsprotokoll gespeicherten Zustände, die Auswahl der zu verwendenden Sensoren, die Sollwerte der zu kontrollierenden physiologischen Daten und Korrekturmaßnahmen enthalten sind, wobei die kontrollierten Daten, die Befolgung der Therapie und die Ergebnisse der Therapien am Plan angehängt sind, der nur durch ausdrückliche Autorisierung des einzelnen Patienten und/oder seiner Pflegeperson zugreifbar ist.

6. System gemäß einem der Ansprüche 1 bis 5, das ein Protokoll-Manager-Modul (20) zur Kontrolle der Warnsignalen und der Ereignisinformationen im Zustandsprotokoll (11; 22) und zur Überwachung der Berichte im Übergabe-protokoll (12; 28; 36) enthält.

7. System gemäß einem der Ansprüche 1 bis 6, das ein Diagnostik-Manager-Modul (30) zur Diagnostizierung des Zustands der einzelnen Patienten mittels des Vergleichs der in Expertencomputerprogrammen gespeicherten Daten umfasst, wobei die Expertencomputerprogramme Diagnoserichtlinien mit den akkumulierten Daten der Wissensdatenbank enthalten.

8. Computerprogrammprodukt, das auf einem in einem Computersystem verwendbaren Medium gespeichert ist, zur Verwaltung und zum Management der Gesundheitsversorgung einer großen Zahl einzelner Patienten, welches einen Softwarecode besitzt, der geeignet ist, das System gemäß Anspruch 1 zur Durchführung nachstehender Schritte zu veranlassen:
- Übertragen, Eingeben und Speichern aller wichtigen physiologischen Daten und Ereignisinformationen der einzelnen Patienten, gemeinsam mit Warnsignalen für den einzelnen Patienten, in ein Zustandsprotokoll (11; 22) einer Zustandskontrollvorrichtung (20),
- Bewerten der Warnsignalen und Ereignisinformationen und Versenden der Warnsignalen und Ereignisinformationen an die beteiligten Pflegepersonen, mit dem Ziel, für den einzelnen Patienten eine Korrekturmaßnahme zu ergreifen und zur Abfrage eines Berichts über die Korrekturmaßnahme und deren Ergebnisse sowie zum Speichern der Berichte in einem Übergabe-protokoll (12; 28) einer Überwachungsvorrichtung (20), und
- Evaluieren von Problemen und der Messwertmuster mittels Vergleichens der Probleme und Muster mit den akkumulierten Informationen in einer Wissensdatenbank (36) einer Evaluierungsvorrichtung (30) zur Auswahl einer geeigneten Korrekturmaßnahme, die den Pflegepersonen empfohlen wird, und zur Abfrage eines Berichts der Pflegepersonen über die Korrekturmaßnahme und deren Ergebnisse sowie zum Eingeben der Berichte in die Wissensdatenbank, wodurch die Wissensdatenbank akkumuliert und vergrößert wird, und
- Übertragen von Warnungen und Empfehlungen mittels eines mit einem Computersystem (1) verbundenen Kommunikationssystems (10; 13; 43; 51) an einen Supervisor, der die Warnungen und Empfehlungen korrigiert und/oder ausgibt, die weiter an die Pflegepersonen geleitet werden, damit diese die empfohlene Maßnahme ergreifen.

## Revendications

1. Système d'entretien et de gestion de soins de santé pour un grand nombre de patients, comprenant un système informatique et un système de communication mis en communication avec le système informatique, dans lequel le système informatique (1) comprend:
- un dispositif de surveillance de l'état (20) comprenant un fichier journal d'état (11 ; 22), destiné à l'entrée et au stockage de toutes les données physiologiques importantes et des informations d'événement de chaque patient ainsi que de signaux d'alarme pour ledit patient,
- un dispositif de supervision (20) comprenant un fichier journal de changements (12 ; 28), destiné à évaluer les signaux d'alarme et les informations d'événement et à transmettre les signaux d'alarme et les informations d'événement aux professionnels de santé concernés afin de déclencher une action corrective pour chaque patient et de demander un rapport sur l'action corrective et ses résultats et d'enregistrer les résultats, et
- un dispositif d'évaluation (30) conçu pour évaluer les problèmes et les schémas des mesures en comparant les problèmes et schémas avec les informations cumulées dans une base de données de connaissances (36), en vue de sélectionner une mesure corrective appropriée à recommander aux professionnels de santé et de demander un rapport des professionnels de santé sur l'action corrective et ses résultats et pour entrer les rapports dans la base de données de connaissances, en cumulant et augmentant ainsi la base de données de connaissances, et
le système de communication (10 ; 13 ; 43 ; 51) est prévu pour transmettre des alarmes et des recommandations à un surveillant afin de corriger et/ou de valider les alarmes et recommandations, qui sont en outre transmises aux professionnels de santé pour qu'ils prennent la mesure recommandée pour le patient en question.

2. Système selon la revendication 1, dans lequel le système de communication (10' ; 13 ; 43 ; 51) comprend des dispositifs pour l'accès à Internet, en particulier des téléphones mobiles (41), organiseurs et ordinateurs personnels avec accès à Internet.

3. Système selon la revendication 1 or 2, dans lequel des capteurs (48 ; 50 ; 52 ; 55 ; 56) sont prévus pour mesurer les données physiologiques du patient et sont connectés au système de communication (10 ; 13 ; 43 ; 51).

4. Système selon l'une des revendications 1 à 3, comprenant un site Web médical stocké dans le système informatique (1), qui contient une base de données séparée contenant toutes les données médicales et l'historique médical de chaque patient et accessible uniquement sur autorisation expresse du patient et/ou de son professionnel de santé.

5. Système selon l'une des revendications 1 à 4 comprenant un plan de santé personnalisé stocké dans le système informatique (1), contenant les prescriptions établies par les médecins pour des médicaments, traitements, pratiques et habitudes de vie du patient en question, un programme de surveillance des pathologies mémorisées dans le fichier journal d'état, le choix des capteurs à utiliser, les seuils des valeurs physiologiques à surveiller et les actions correctives, les données surveillées, l'observance des traitements et le résultat des traitements étant joints au plan, qui est accessible uniquement sur autorisation expresse du patient et/ou de son professionnel de santé.

6. Système selon l'une des revendications 1 à 5 comprenant un module de gestionnaire de fichier journal (20) pour surveiller les signaux d'alarme et les informations d'événement dans le fichier journal d'état (11 ; 22) et superviser les rapports dans le fichier journal de changements (12 ; 28 ; 36).

7. Système selon l'une des revendications 1 à 6, comprenant un module de gestionnaire de diagnostic (30) destiné à diagnostiquer l'état de chaque patient au moyen d'une comparaison des données mémorisées dans des programmes informatiques experts contenant des instructions de diagnostic avec les données cumulées de la base de données de connaissances.

8. Programme informatique stocké sur un support utilisable dans un système informatique pour l'entretien et la gestion de soins de santé pour un grand nombre de patients, comprenant un code de logiciel apte à faire exécuter les étapes suivantes au système selon la revendication 1 :
- transmission, saisie et stockage de toutes les données physiologiques importantes et des informations d'événement de chaque patient avec les signaux d'alarme pour ledit patient dans un fichier journal d'état (11; 22) d'un dispositif de surveillance de l'état (20),
- évaluation des signaux d'alarme et des informations d'événement et transmission des signaux d'alarme et des informations d'événement à des professionnels de santé concernés afin de déclencher une action corrective pour le patient et de demander un rapport sur l'action corrective et de stocker les rapports dans un fichier journal de changements (12 ; 28) d'un dispositif de supervision (20), et
- évaluation des problèmes et des schémas des mesures par comparaison des problèmes et schémas avec les informations cumulées dans une base de données de connaissances (36) d'un dispositif d'évaluation (30), en sélectionnant l'action corrective appropriée à recommander aux professionnels de santé, et demande d'un rapport des professionnels de santé sur l'action corrective et ses résultats et saisie des rapports dans la base de données de connaissances, en cumulant et étendant ainsi la base de données de connaissances, et
- transmission d'alarmes et de recommandations au moyen d'un système de communication (10 ; 13 ; 43 ; 51) connecté au système informatique (1) à un régulateur qui corrige et/ou valide les alarmes et recommandations qui sont ensuite transmises aux professionnels de santé pour qu'ils prennent la mesure recommandée.
